# EUROPEAN PATENT APPLICATION

(11) **EP 2 502 984 A1**
(43) Date of publication of application: **26.09.2012**
(21) Application number: 11305282.3
(22) Date of filing: 15.03.2011
(51) Int. Cl.: C12N 5/00, C12N 5/09, G01N 33/50

(54) **Multicellular aggregates of lymphomatous cells and method for preparing thereof**

(71) Applicant: INSERM (Institut National de la Santé et de la Recherche Médicale), 75013 Paris (FR)
(72) Inventor: The designation of the inventor has not yet been filed
(74) Representative: Cabinet Plasseraud

(57) **Abstract**

The present invention provides a method for preparing a multicellular aggregate of lymphomatous cells (MALC). The major principle of the invention is a modification of the "hanging drop" method by introducing a gelling agent during drop preparation. This technique provide a reliable method for obtaining stiff 3D culture permitting cell biology analyses, immunohistochemistry studies, as well as drug distribution and efficacy measurement. Through the possibility to introduce matrix proteins and non-tumour cells, this model offers also the opportunity to recreate lymphoma microenvironment.

## Description

### FIELD OF THE INVENTION

The present disclosure provides multicellular aggregates of lymphomatous cells (MALC).

### BACKGROUND OF THE INVENTION

Classically, most cell culture experiments have been performed under monocellular cells suspension, in 2D conditions. As disclosed by Keiran et al. ( "Life isn't flat: taking cancer biology to the next generation ", In vitro Cell. Dev. Biol.- Animal 42:242-247), culturing in 2D conditions is a situation that ignores that tumours are in fact "an organ". Indeed, cells in the human body grow within an organized 3D matrix, surrounded by other cells. The behavior of individual cells is controlled through their interactions with their immediate neighbours and the extracellular matrix. Keiran further emphasizes that three-dimensional models provide the opportunity to explore the behavior of the cells in a setting that attempts to reproduce the dynamics of a tumour interaction with its natural environment. As a result, there are a growing number of studies which report differences in phenotype, cellular signalling, cell migration, and drug responses when the same cells are grown under 2D or 3D culture conditions. One potential application of these techniques is anticancer drug discovery, which has long been hampered by the lack of good preclinical models. Compounds with good anti-tumour activity in 2D cell culture models often fail to translate into the clinic.

To overcome the shortcomings of the 2D culture, many studies in the cancer field are based on xenotransplantation. While being a more accurate model than 2D culture, xenotransplantation is an expensive technique, which is not appropriate for large scale experiments, sometimes lacks reproducibility, and requires the use of animals, which can be an ethical problem.

Del Duca et al ( "Spheroid preparation from hanging drops: characterization, of a model of brain tumour invasion ", Journal of Neuro-Oncology 67:295-303, 2004) disclosed a technique of multicellular aggregates preparation by "hanging drop" providing a three-dimensional *in vitro* model of brain tumour. Such a technique results in the formation of multicellular aggregates of adherent cells, called spheroid. However, this method is suitable for adherent cells like epithelial cells and not for non-adherent cells like hematopoietic cells.

There is hence still a need for a 3D *in vitro* model for non adherent cells such as lymphoma cell lines.

### SUMMARY OF THE INVENTION

The inventors have adapted the "hanging drop" technique, previously described by Del Duca et al., to non-adherent cells in order to obtain 3D *in vitro* models and to recreate the behavior of a human lymphoma *in vivo.*

The present invention provides a method for preparing a multicellular aggregate of lymphomatous cells comprising the step of incubating a drop containing lymphomatous cells in a culture medium, wherein said drop is hanging from a support and said culture medium comprises a gelling agent.

The invention also relates to a multicellular aggregate of lymphomatous cell obtainable by said method.

The invention further provides a method for screening compounds for treating and/or preventing lymphoma comprising the following steps:
a) providing a multicellular aggregate of lymphomatous cells according to the invention ;
b) adding the compound to be screened ; and
c) selecting the compound which inhibits the growth of said multicellular aggregate of lymphomatous cells and/or leads to the death of said lymphomatous cells.

### DETAILED DESCRIPTION OF THE INVENTION

As used herein, the term "multicellular aggregate" in all its grammatical forms, refers to an aggregate or assembly of cells cultured in a 3D growth as opposed to growth as a monolayer or 2D culture. The term multicellular aggregate does not necessarily imply that the aggregate is a geometric sphere. Indeed, the aggregate are organised in mass whether or not they displayed a spherical morphology. According to the invention, the multicellular aggregate of lymphomatous cells contains at least 25 aggregated cells, preferably from 25 to 10⁶ cells, preferably from 25 to 10³, preferably from 25 to 500 cells, preferably from 50 to 150, and most preferably about 100 cells. However, the number of cells is a linear function of time over the culture duration, which is comprised between 1 and 35 days. Typically, the multicellular aggregate of lymphomatous cells has a volume comprised between 0.1 and 500 mm³, between 0.1 and 400 mm³, between 0.1 and 300 mm³, between 50 and 300 mm³, preferably between 0.1 and 200 mm³.

As used herein the term "constituted multicellular aggregate of lymphomatous cells" refers to a multicellular aggregate containing 10⁴ to 2.10⁶ lymphomatous cells with a volume comprised between 0.1 and 500 mm³.

### Method for preparing a multicellular aggregate of lymphomatous cells

The present invention provides a method for preparing a multicellular aggregate of lymphomatous cells comprising the step of incubating a drop comprising a lymphomatous cell in a culture medium, wherein said drop is hanging from a support, and said culture medium comprises a gelling agent.

Typically, the hanging drop is incubated for a time period sufficient to obtain an aggregate of cells.

As used herein, the term "lymphomatous cell" refers to a cell of a tumour originated from lymphoid tissue.

The expression lymphomatous cell encompasses "lymphomatous cells lines" and "lymphomatous primary cell lines".

Examples of lymphomatous cell lines are follicular lymphoma cell line, Burkitt lymphoma cell line, mantle lymphoma cell line, B chronic lymphocytic leukemia cell line, diffuse large B cell lymphoma cell line, T cell lymphoma cell line, and by extension multiple myeloma cell line.

Examples of the lymphomatous primary cells are follicular lymphoma, Burkitt lymphoma, mantle cell lymphoma, B chronic lymphocytic leukemia, diffuse large B cell lymphoma, T cell lymphoma, and multiple myeloma.

Examples of gelling agent useful in the present invention include hydrophilic cellulose-based gelling agent such as cellulose, cellulose derivative selected from the group comprising methylcellulose, carboxycellulose, carboxymethylcellulose, hydroxypropylmethylcellulose, hydroxyethylcellulose, hydroxypropylcellulose; xanthan gum, guar gum, carrageenan, gelatine; and mixtures thereof. Preferably, the gelling agent is a hydrophilic cellulose-based gelling agent. Most preferably, the gelling agent is methylcellulose.

The man skilled in the art can refer to the teachings of Del Duca to implement the present invention.

The main principle of the invention is disclosed hereafter.

In a first step, lymphomatous cells are cultured in a classical culture medium. When the cells become confluent, cells are resuspended into a culture medium comprising a gelling agent, for example methylcellulose.

In a second step, a drop of lymphomatous cell suspension is placed onto the lid of a first dish.

In a third step, the lid of the first dish is inverted over said first dish containing a culture medium so as to form a hanging drop of lymphomatous cells suspension. Cell containing drop is then incubated for a various time period depending on the nature of the cells.

In a fourth step, the resulting multicellular aggregates of lymphomatous cells in the hanging drop are transferred to a second dish and are incubated.

In the following, the preferred embodiments of the invention will be described step by step.

In a first step, lymphomatous cells are cultured in a classical medium. When the cells become confluent, cells are resuspended in a culture medium comprising a gelling agent.

The determination of condition of culture of lymphomatous cells in a classical culture conditions are determined by general knowledge of the man skilled in the art. Typically, the culture is maintained at 37°C in a humidified atmosphere of 5% carbon dioxide.

When the cells become confluent (about 1 million cells /ml as measured by automated cell counter), lymphomatous cells are diluted into a culture medium comprising a gelling agent. Typically, the concentration of cells in said cell suspension is from 10³ to 10⁸ cells/ml, from 5.10³ to 5.10⁷ cells/ml, from 10⁴ to 10⁵ cells/ml, preferably from 2.10⁵ to 5. 10⁵, and most preferably about 5.10⁵ cells/ml.

Suitable culture mediums are well known by the man skilled in the art. Typically, such culture medium are enriched with fetal calf serum. Non limiting examples of culture medium comprise Dulbecco's Modified Eagle Medium (DMEM) and Roswell Park Memorial Institute medium (RPMI). For example, for the preparation of multicellular aggregates of lymphomatous cells from RL follicular cell lines, RPMI may be used.

Typically, the gelling agent is present in the medium in a range from 0.1 to 10% v/v, from 0.1 to 3% v/v, from 0.5 to 2% v/v, preferably from 0.5 to 1% v/v, and most preferably about 1% v/v. The concentration of the gelling agent in volume-volume percentage (% v/v) refers to the volume of gelling agent on the volume of the resulting medium.

In a second step, a drop of lymphomatous cell suspension is placed onto the lid of a first dish. Typically, the drop placed onto the lid of a first dish has a volume in a range from 1 µl to 30 µl, 10 µl to 30 µl, 10 µl to 20 µl, preferably about 20 µl.

In a third step, the lid of the first dish is inverted over said first dish containing a culture medium so as to form a hanging drop of lymphomatous cells suspension. Said first dish contains a culture medium from 0.5 ml to 3 ml, preferably from 0.5 to 2 ml, and most preferably about 1 ml of culture medium.

The hanging drop is then incubated. The gravitational force executed in the cell in the hanging drop causes sedimentation, resulting in the formation of multicellular aggregates of lymphomatous cells. The hanging drop is incubated for a time period sufficient to obtain aggregates of cells.

Typically, the hanging drop is incubated for a time period comprised between 8 hours and 7 days, between 8 and 96 hours, 16 and 72 hours, 16 and 48 hours, 18 and 36 hours, 20 and 28 hours, 22 and 26 hours, and preferably about 24 hours.

In a fourth step, the resulting multicellular aggregates of lymphomatous cells in the hanging drop are transferred to a second dish and then are incubated in culture condition determined by the man skilled in the art. Such culture conditions depend, *inter alia,* on the type of cultured cells and the concentration of the cells in the drop. All the cells cultured in the second dish are contained the multicellular aggregates of lymphomatous cells as observed by the microscope. There is almost no cell (less then 1%) in the surrounding medium indicating that the multicellular aggregates of lymphomatous cells displays attractive properties for surrounding cells.

The cellular aggregates may be transferred to said second dish by any technique known by the man skilled in the art, including transferring by drop fusion or harvesting using a Pasteur pipette under dissecting microscope.

Typically, the second dish is a dish base-coated with a solid culture medium and filled with a culture medium. Suitable solid culture mediums are well known by the man skilled in the art. Non limiting examples of solid growth medium comprise agar, agarose and agar-agar. Typically, Agar concentrations are comprised between 0.5% and 2% g/ml and preferably 1% g/ml. The concentration of agar, agar-agar or agarose in g/ml percentage (% g/ml) refers to the mass of agar, agar-agar or agarose on the volume of the water used for the preparation of the solid growth medium.

In one embodiment of the invention, the method according to the invention comprises in the first step, the culture of lymphomatous cells with non-tumour cells. Non tumour cells can be added to lymphomatous cells in the classical culture medium or in the resuspension of lymphomatous cells in the culture medium comprising a gelling agent, preferably methylcellulose.

Alternatively, the method of the invention comprises a step of incubating the multicellular aggregates of lymphomatous cells with non-tumour cells after the fourth step, i.e. the transfer of multicellular aggregates of lymphomatous cells.

Typically, non tumour cells are selected from the group consisting of immune cells, endothelial cells, fibroblasts, stroma cells and mixtures thereof.

Indeed, the non tumour cells may either be cultured with lymphomatous cells in the culture medium comprising a gelling agent, preferably methylcellulose, either be incorporated by the multicellular aggregates of lymphomatous cells.

In another embodiment of the invention, the method according to the invention comprises in the first step, culture of lymphomatous cells with matrix components. Matrix component can be added to lymphomatous cells in the classical culture medium or in the resuspension of lymphomatous cells in the culture medium comprising a gelling agent, preferably methylcellulose. Alternatively, the method of the invention comprises a step of incubating the multicellular aggregates of lymphomatous cells with matrix components after the fourth step, i.e. the transfer of multicellular aggregates of lymphomatous cells.

Typically, said matrix components comprise proteins selected from the group consisting of fibronectin, laminin, tenascin, collagen, gelatin, and mixtures thereof.

Example of mixture of matrix components is Matrigel. As used herein, "Matrigel" refers to BD Matrigel™ Basement Membrane Matrix, which is a commercial preparation of basement membrane produced by Engelbreth-Holm-Swarm tumour cells and containing extracellular matrix components such as laminin. Matrigel is available commercially through Becton, Dickinson and Company (Franklin Lakes, NJ).

### Multicellular aggregates of lymphomatous cells (MALC)

The invention also relates to multicellular aggregates of lymphomatous cells obtainable by the method described above.

Typically, such a multicellular aggregate contains, in number of cells, from 80 to 99%, from 90 to 99%, preferably about 99% of lymphomatous cells.

One major advantage of multicellular aggregates of lymphomatous cells according to the invention is to provide materials for cell biology analyses. For example, thanks to its sufficient stiffness, multicellular aggregates of lymphomatous cells may be used for performing regular immunohistochemistry according to standard techniques well know by the man skilled in the art.

The multicellular aggregates of lymphomatous cells according to the invention are resistant to gentle shaking. However, it is possible to obtain a complete dislocation of the multicellular aggregate of lymphomatous cells under mechanical pressure using a pipette without altering cell integrity and future growth capacity. This property allows further cellular or biochemical analyses.

The multicellular aggregate of lymphomatous cells growth is a linear function of time from day 1 to day 30. During this period the cell doubling time is constant and could be calculated (18 hours). After day 30, the multicellular aggregate of lymphomatous cells growth slows down and typically the multicellular aggregate of lymphomatous cells volume decreases after day 40.

Multicellular aggregates of lymphomatous cells display identical phenotype than the original cell lines used for its constitution. For example multicellular aggregates of lymphomatous cells from RL follicular cell lines display a typical follicular lymphoma cell immunophenotype profile (CD20+, CD10+, CD5-) as measured by cytometry analysis of multicellular aggregates of lymphomatous cells after dislocation.

In one embodiment of the invention, the multicellular aggregate of lymphomatous cells further comprises non tumour cells.

Typically, said non tumour cells are selected from the group consisting of immune cells, endothelial cells, fibroblasts, stroma cells and mixtures thereof.

Typically, the non tumour cells represents from 1% to 99%, preferably from 10% to 80%, preferably from 20% to 70%, preferably from 30% to 60%, preferably from 40% to 50%, most preferably from 1% to 50% by number of cells of the multicellular aggregate of lymphomatous cells.

In still another embodiment of the invention, the multicellular aggregate of lymphomatous cells further comprises matrix components.

Typically, said matrix components are selected from the group consisting of fibronectin, laminin, tenascin, collagen, gelatin, and mixtures thereof.

In still another embodiment of the invention, the multicellular aggregate of lymphomatous cells further comprises non tumour cells and matrix components.

Such multicellular aggregates of lymphomatous cells further comprising non-tumour cells and/or matrix components are a more accurate model of the complex composition of a tumour and provides conditions for reproducing *in vitro* the complex network of the *in vivo* microenvironment.

Indeed, addition of non-tumour cells and/or matrix components in the multicellular aggregates of lymphomatous cells provides conditions mimicking the conditions of *in vivo* tumours, in which matrix proteins accumulate. For example, such a model may be used to mimic infiltration of immune cells in a tumour, and to reproduce the interaction between lymphomatous cells, as well as lymphomatous cells-non tumour cells interactions.

### Screening anticancer drug candidates

The present invention thus further provides a method for screening compounds (i.e. drug candidates) for treating and/or preventing lymphoma comprising the following steps:
a) providing a multicellular aggregate of lymphomatous cells according to the invention ;
b) adding the compound to be screened ;
c) selecting the compound which inhibits the growth of said multicellular aggregate of lymphomatous cells and/or leads to the death of said lymphomatous cells.

The multicellular aggregate of lymphomatous cells according to the invention can be used for screening either conventional compound such as antracyclines or bioacting agents such as monoclonal antibodies. 3D culture offers conditions for drug screening more relevant to the in vivo situation compared to the 2D culture. Indeed, 3D culture integrates specific parameters such as drug diffusion (Minchinton et al, Nat Rev Cancer, 2006) and possible changes in intracellular signalling (Pickl et al., Oncogene 2009), all greatly contributing to drug efficacy.

The man skilled in the art will be aware of standard methods for implementing this method.

In the following, the invention will be illustrated by means of the following examples as well as the figures.

### FIGURES LEGENDS

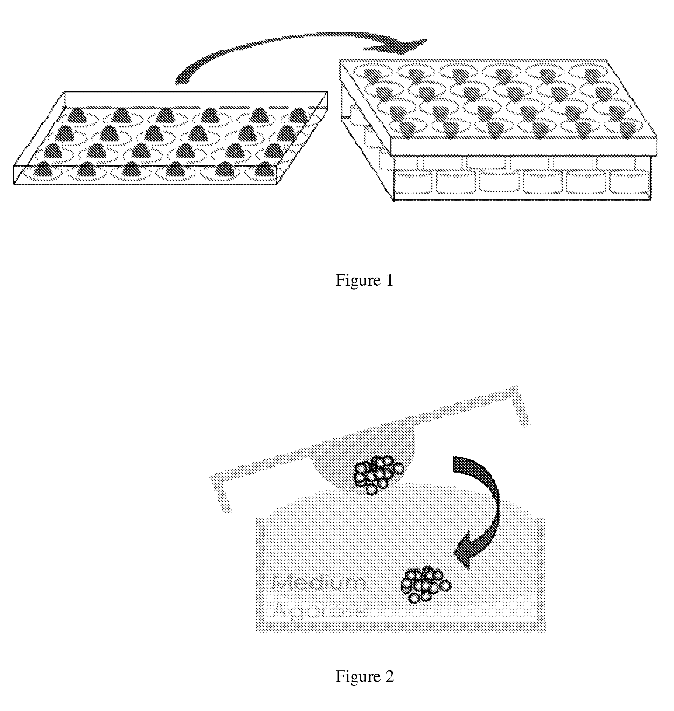

**Figure 1****:** Cells are cultured on the lid of a culture dish and are then inverted over plate containing 1 ml medium.
**Figure 2****:** Hanging drop cultures are incubated for 24h. The resulting cellular aggregate is transferred into the plat B by drop fusion.
**Figure 3****:** Multicellular aggregates of lymphomatous cells (MALC) expansion (volume and cell number).
**Figure 4****:** Immunohistochemistry using MALC : example of anti CD10 staining.
**Figure 5****:** Capacity of MALC to incorporate exogenously added protein matrix component such as collagen I, fibronectin, gelatin, and mixture of all these components (Matrigel)
**Figure 6****:** Use of MALC for testing antracyclines (Daunorubicin): a) drug incorporation into cells; b) effect on MALC volume.
**Figure 7****:** Impact of Rituximab on MALC growth.

### EXAMPLE

### Materials and methods

### Cell lines

RL are transformed Follicular Lymphoma cell lines carrying the t(14;18). There were obtained from the ATCC (American Type Cell Collection) (Rockville, MD, USA). Cells were cultured at 37°C in 5% CO2 in RPMI supplemented with 10% fetal calf serum (FCS), glutamine (2 mM), streptomycin (10 µg/mL) and penicillin (200 U/mL) (Invitrogen, Cergy Pontoise, France).

### MALC volume expansion correlates with tumour cell number

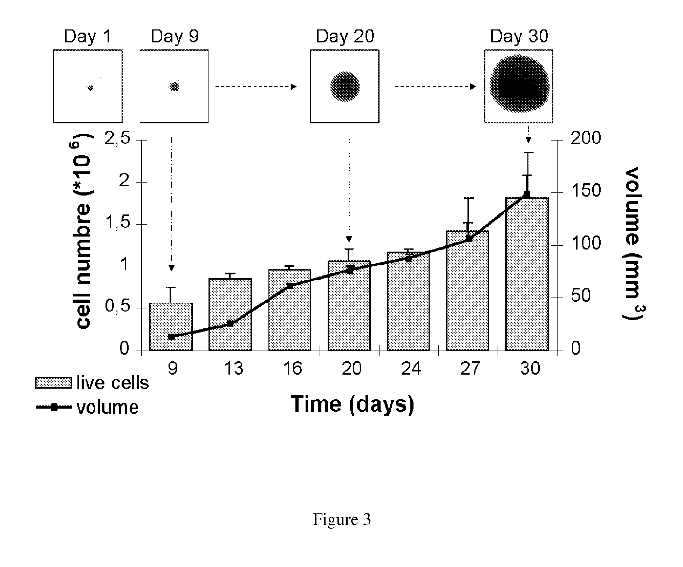

MALC resembles to a growing size oblate sphere as shown in Figure 3, MALC volume ranging between 0.1 mm³ at day 1 and 200 mm³ at day 30.

The inventors have here shown that MALC volume expansion correlates with tumour cell number. Cell density (number of viable cells per MALC volume) increases as a linear function of time. (Figure 3)

### CD10 expression

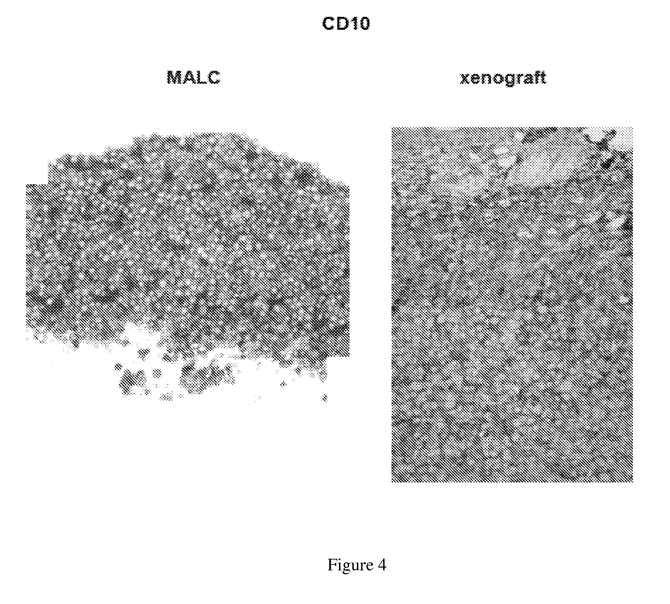

The inventors have investigated MALC-contained cell phenotype (Figure 4, CD 10 staining). In 2D culture, RL cells displayed an immunophenotype characteristic of follicular lymphoma, CD20+, CD10+, CD5neg. This phenotype was unchanged in MALC cells compared to 2D culture conditions and xenograft.

### MALC incorporates exogenously added protein matrix component

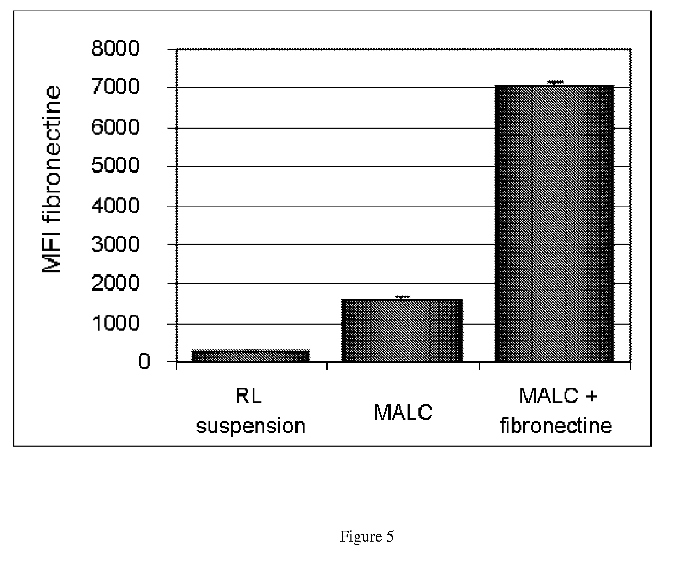

The inventors investigated at which extent, MALC incorporates exogenously added protein matrix components such as collagen I, fibronectin, gelatin, and mixtures of all these components (Matrigel). Figure 5 shows that MALC does incorporate fibronectin. Addition of matrix proteins mimics the conditions of human tumours in which one assume that most of these proteins originate from extra tumour sources, such as tumour associated fibroblasts.

This model can be used to measure the influence of matrix proteins on biodiffusion of drugs or antibodies into MALC.

### Response to Daunorubicin

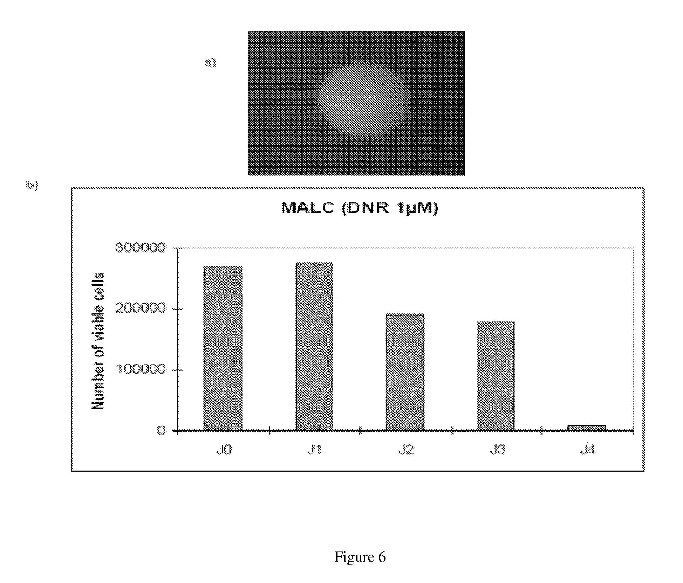

The inventors have investigated the impact of Daunorubicin on MALC (1µM). Figure 6a shows Daunorubicin incorporation after 24hours incubation (as visualized by fluorescence microscopy tacking advantage of drug autofluorescence). Figure 6b shows time-dependent effect of drug on MALC volume. The drug has been added (1µM) to 7 days constituted MALC.

The inventors have thus showed the intracellular incorporation of drugs by MALC and the effect thereof on MALC growth.

### Response to Rituximab

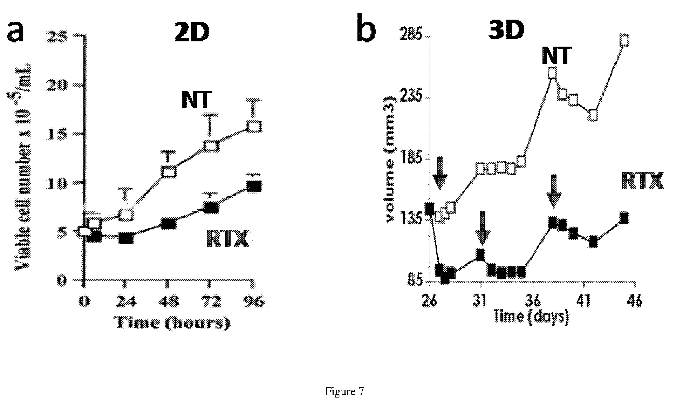

The inventors have investigated the impact of Rituximab, a monoclonal antibody directed against CD20 and largely used in the treatment of lymphoma. The inventors found out that Rituximab almost totally blocked RL proliferation cultured as MALC (Figure 7).

The effect of Rituximab on MALC resembles to that described for human xenograft in mice (Dalle S et al., Clin Cancer Res. 2009). These results indicate that Rituximab was much more efficient in the 3D culture system, compared to the 2D culture system. This observation was even more unexpected based on reduced CD20 expression in MALC, compared to the 2D culture conditions.

### References

Throughout this application, various references describe the state of the art to which this invention pertains. The disclosures of these references are hereby incorporated by reference into the present disclosure.

## Claims

1. A method for preparing a multicellular aggregate of lymphomatous cells comprising the step of incubating a drop comprising lymphomatous cells in a culture medium, wherein said drop is hanging from a support and said culture medium comprises a gelling agent.

2. The method according to claim 1, wherein said gelling agent is an hydrophilic cellulose-based gelling agent.

3. The method according to claim 1 or 2, wherein said gelling agent is chosen from the group consisting of cellulose, methylcellulose, carboxycellulose, carboxymethylcellulose, hydroxypropylmethylcellulose, hydroxyethylcellulose, hydroxypropylcellulose.

4. The method according to any one of claims 1 to 3, wherein said gelling agent is methylcellulose.

5. The method according to any one of claims 1 to 4, wherein said gelling agent is present in said culture medium in a range from 0.1 to 10% v/v, preferably from 0.1 to 3% v/v, preferably from 0.5 to 2% v/v, preferably from 0.5 to 1% v/v, and most preferably about 1% v/v.

6. The method according to any one of claims 1 to 4, wherein said drop is incubated for a time period comprised between 8 hours and 7 days, preferably between 8 and 96 hours, preferably 16 and 72 hours, preferably 16 and 48 hours, preferably 18 and 36 hours, preferably 20 and 28 hours, preferably 22 and 26 hours, and most preferably about 24 hours.

7. The method according to any one of claims 1 to 6, wherein said drop comprising lymphomatous cells further comprises non-tumour cells selected from the group consisting of immune cells, endothelial cells, fibroblasts, and mixtures thereof.

8. A multicellular aggregate of lymphomatous cells obtainable by the method as defined in any one of claims 1 to 7.

9. The multicellular aggregate of lymphomatous cells according to claim 8, wherein said multicellular aggregate comprises at least 25 aggregated cells, preferably from 25 to 10⁶ cells, preferably from 25 to 10³, preferably from 25 to 500 cells, preferably from 50 to 150, and most preferably about 100 cells.

10. The multicellular aggregate of lymphomatous cells according to claim 8 or 9, wherein said multicellular aggregate has a volume comprised between 0.1 and 500 mm³, between 0.1 and 400 mm³, between 0.1 and 300 mm³, between 50 and 300 mm³, preferably between 0.1 and 200 mm³.

11. The multicellular aggregate of lymphomatous cells according to any one of claims 8 to 10, wherein said multicellular aggregate comprises, in number of cells from 80 to 99%, preferably from 90 to 99%, most preferably about 99% of lymphomatous cells.

12. The multicellular aggregate of lymphomatous cells according to any one of claims 8 to 11, wherein said multicellular aggregate further comprises non tumour cells selected from the group consisting of immune cells, endothelial cells, fibroblasts and mixtures thereof.

13. The multicellular aggregate of lymphomatous cells according to any one of claims 8 to 12, wherein said multicellular aggregate further comprises matrix components selected from the group consisting of fibronectin, laminin, tenascin, collagen, gelatin and mixtures thereof.

14. A method for screening compounds for treating and/or preventing lymphoma comprising the following steps:
a) providing a multicellular aggregate of lymphomatous cells as defined in claims 9 to 13;
b) adding the compound to be screened; and
c) selecting the compound which inhibits the growth of said multicellular aggregate of lymphomatous cells and/or leads to the death of said lymphomatous cells.

15. The method according to claim 14, wherein said compound is a monoclonal antibody.
